# EUROPÄISCHE PATENTSCHRIFT

(11) **EP 1 371 976 B1**
(45) Veröffentlichungstag und Bekanntmachung des Hinweises auf die Patenterteilung: **05.04.2006**
(21) Anmeldenummer: 03012351.7
(22) Anmeldetag: 30.05.2003
(51) Int. Cl.: G01N 33/00, G01N 27/28

(54) **Messgassonde zur Messung von Gasen in einer heissen und staubhaltigen Gasatmosphäre**
Gas detector for the detection of gases in hot and dusty atmospheres
Capteur de gaz pour la mesure de gaz dans une atmosphère chaude et poussiéreuse

(30) Priorität: 10.06.2002 DE 10225827
(43) Veröffentlichungstag der Anmeldung: 17.12.2003
(73) Patentinhaber: Sobotta GmbH Sondermaschinenbau, 53819 Neunkirchen-Seelscheid (DE)
(72) Erfinder: Gumprecht, Fred, 51709 Marienheide (DE); Sobotta, Kurt, 53819 Neunkirchen-Seelscheid (DE)
(74) Vertreter: Radünz, Ingo

(56) Entgegenhaltungen:
- DD-A- 111 248
- DD-A- 301 963
- DE-A- 2 302 981
- DE-A- 3 545 491
- DE-A- 19 947 655
- DE-C- 3 733 355
- US-A- 3 486 382
- US-A- 4 466 880

## Beschreibung

Die Erfindung betrifft eine Messgassonde für die In-Situ-Messung von gasförmigen Rauchgasbestandteilen in einer heißen, staubhaltigen Gasatmosphäre.

Die Betreiber der Feuerungsanlagen von Kraftwerken, Müllverbrennungsanlagen, Glasschmelzen oder Zementdrehöfen und anderen sind nicht zuletzt aus Umweltgründen gehalten, eine einwandfreie Verbrennung möglichst mit Hilfe einer Regelung der Feuerung zu gewährleisten. Um die vorgeschriebenen Umweltdaten einhalten zu können, ist ständig unter anderem die Sauerstoffkonzentration im Rauchgas zu überwachen.

Es sind Messverfahren und Messanordnungen zur Bestimmung der gasförmigen Rauchgasbestandteilen bekannt, bei denen mittels wassergekühlter Gasentnahmesonden das Messgas aus dem Feuerraum angesaugt und über temperierte Schläuche und weite Wege einem Gas-Analysengerät zugeführt wird. Der Messvorgang dauert 3 bis 4 Minuten vom Zeitpunkt der Ansaugung her, was für eine Regelung des Feuerungsprozesses wesentlich zu lang ist.

In staubbeladenen Feuerungen von z. B. Zementdrehöfen besteht an den bisher bekannten Sonden die Gefahr des Anbackens von Staub im Innern des Messraumes (Rohmehlansatz). Um diese Sonden wieder von Anbackungen freizubekommen, sind erhebliche Demontage- und Montagearbeiten sowie Wartungsarbeiten nötig. Diese Arbeiten haben einen längeren Ausfall der Messung zur Folge.

Der Erfindung liegt die Aufgabe zugrunde, eine in einer heißen, staubbeladenen Gasatmosphäre einzusetzende Messgassonde für eine In-Situ-Messung zu schaffen, die verzögerungs- und weitgehend wartungsfrei arbeiten kann.

Die Aufgabe wird erfindungsgemäß durch die Merkmale des Anspruches 1 gelöst. Vorteilhafte Ausgestaltungen der Erfindung sind Gegenstand der Unteransprüche.

In der erfindungsgemäßen Messgassonde liegt der Sensor geschützt vor den Einflüssen der heißen, staubhaltigen Gasatmosphäre. Dadurch wird es möglich, auch unter diesen ungünstigen Betriebsbedingungen eine In-Situ-Messung durchführen zu können. Die oszillierende Axial- und Drehbewegung verhindert ein Anbacken von Staub auf und in der Messgassonde und an dem Durchtritt der Messgassonde durch die Wand der Feuerung. Außerdem kann der Staub ausgestoßen werden, der sich vor der Messsonde im Sondenkopf ansammelt .

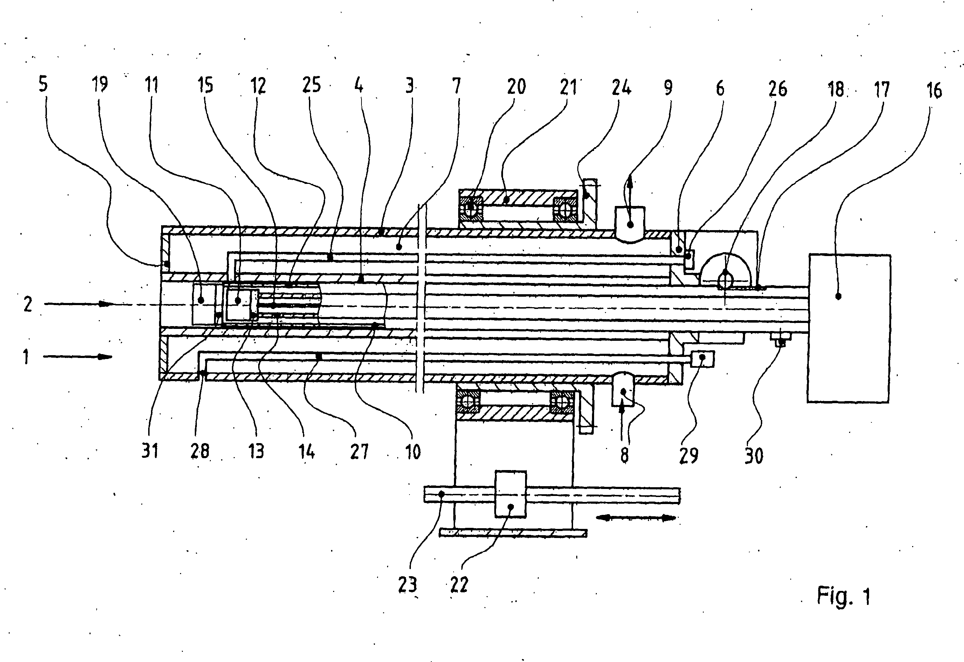

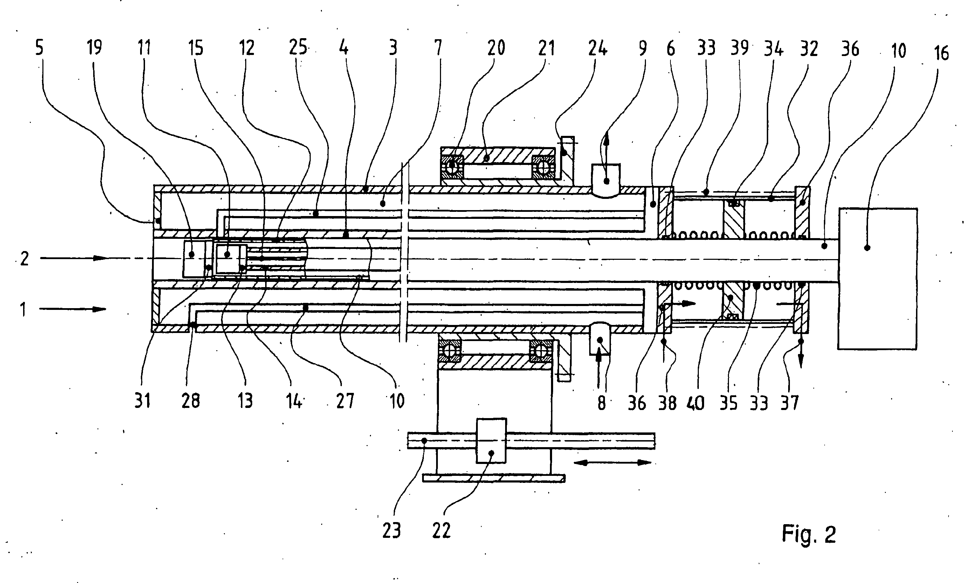

Mehrere Ausführungsbeispiele der Erfindung sind in der Zeichnung dargestellt und werden im Folgenden näher erläutert. Die Fig. 1 und 2 Zeichnung geben jeweils den Längsschnitt durch eine Messgassonde wieder.

Die dargestellte Messgassonde ist für die Bestimmung von gasförmigen Bestandteilen im Rauchgas, insbesondere des Gehaltes an Sauerstoff, Stickoxid (NOₓ) und Kohlenmonoxid (CO) bestimmt und wird an Feuerungen mit einer sehr staubhaltigen und heißen Gasatmosphäre von bis zu 1500°C eingesetzt. Ein bevorzugter Anwendungsfall ist der Einsatz an Zementdrehrohröfen.

Die Messgassonde weist eine an den Messort angepasste Baulänge zwischen 2 m bis 4,5 m auf. Sie besteht aus einem wassergekühlten Sondenkörper 1 und einer in dem Sondenkörper 1 angeordneten Messsonde 2.

Der Sondenkörper 1 besteht aus einem Außenmantel 3 und einen darin mit radialen Abstand konzentrisch oder wahlweise exzentrisch angeordneten Innenmantel 4. An den einander gegenüber liegenden Enden sind der Außenmantel 3 und der Innenmantel 4 durch einen Frontdeckel 5 und einen Abschlussdeckel 6 zur Bildung eines dichten, von Kühlwasser durchströmten Zwischenraumes 7 verbunden. Der Außenmantel 3 ist an seinem rückwärtigen Ende mit einem Anschluss für den Kühlwassereinlauf 8 und mit einem Anschluss für den Kühlwasserauslauf 9 versehen. Der Kühlwassereinlauf 8 befindet sich in der 6-Uhr-Stellung und der Kühlwasserauslauf 9 in der 12-Uhr-Stellung. In dem Zwischenraum 7 angeordnete Leitbleche sorgen für einen Durchlauf des Kühlwassers durch den Zwischenraum 7.

Die Messsonde 2 ist in dem Innenmantel 4 des Sondenkörpers 1 angeordnet. Sie weist ein äußeres, aus Edelstahl gefertigtes Rohr 10 und ein darin angeordnetes Innenrohr 14 auf. Das äußere Rohr 10 ist an seinem vorderen Ende mit einem rohrförmigen, aus einem Sinterwerkstoff bestehenden Filter 12 verbunden. Das Filter 12 umschließt eine Messkammer 11, in der ein oder mehrere Sensoren 13 zur Bestimmung der gasförmigen Bestandteile des Rauchgases angeordnet sind. Es kann beispielsweise jeweils ein Sensor zur Bestimmung des Gehaltes an Sauerstoff (O₂), Stickoxid (NOₓ) und an Kohlenmonoxid (CO) vorgesehen werden. Die Sensoren 13 sind mit dem äußeren Rohr 10 der Messsonde 1 verbunden. Das Messgas tritt durch den Ringspalt zwischen dem Sondenkörper 1 und der Messsonde 2 ein und gelangt durch das Filter 12 in die Messkammer 11, wo es mit den Sensoren 13 in Berührung kommt. Die treibende Kraft für das Ansaugen des Messgases ist ein Unterduck, der in der Messsonde 2 von außen erzeugt wird.

Durch das Innenrohr 14 sind elektrische Leitungen 15 zu einem Anschlussgehäuse 16 geführt. Das Anschlussgehäuse 16 ist an dem aus dem Sonderkörper herausragenden Ende des äußeren Rohres 10 befestigt. Über die elektrischen Leitung werden die elektrischen Signale der Sensoren 13 nach außen geleitet.

Das Filter 12 ist mit einem Stößel 31 verbunden, der vorne mit einer Ausstoßplatte 19 versehen ist. Der Stößel 31 begrenzt die Messkammer 11 zur Sondenspitze hin.

Die Messsonde 2 ist axial beweglich in dem Innenmantel 4 des Sondenkörpers 1 angeordnet. Auf dem aus dem Abschlussdeckel 6 des Sondenkörpers 1 herausragenden Ende des äußeren Rohres 10 ist eine Zahnstange 17 befestigt. In die Zahnstange 17 greift das Zahnrad eines Antriebsmotors 18 ein. Der Antriebsmotor 18 führt über eine entsprechende Steuerung eine oszillierende Bewegung aus, durch die die Messsonde 2 um etwa 100 mm vor- und rückwärts bewegt wird. Diese axiale alternierende Bewegung hat den Zweck, pärtikelförmige Ansammlungen vor der Messkammer 11 abzuwerfen, die den Messvorgang behindern würden. Das Abwerfen geschieht durch die Ausstoßplatte 19.

Ein anderer Antrieb für die oszillierende Axialbewegung der Messsonde 2 ist in der Fig. 2 dargestellt. Dieser Antrieb besteht aus einer mit Druckluft betriebenen Kolben-Zylinder-Anordnung, die an dem Sondenkörper 1 befestigt ist. Im Einzelnen enthält die Kolben-Zylinder-Anordnung einen zylindrischen Kolben 40, der auf dem aus dem Sondenkörper 1 herausragenden Teil des äußeren Rohres 10 der Messsonde 2 fest aufgeklemmt ist. Der Kolben ist in einem Zylindermantel 32 axial geführt, der beiderseits des Kolbens 40 durch je einen Zylinderdeckel 36 verschlossen ist. Die Zylinderdeckel 36 sind über Zugstangen 39 miteinander und der dem Sondenkörper 1 zugewandte Zylinderdeckel 36 ist mit dem Abschlussdeckel 6 des Sondenkörpers 1 verbunden.

Beiderseits des Kolbens 40 ist je eine Dämpfungsfeder 35 vorgesehen, die jeweils auf dem Kolben 40 und auf einem der Zylinderdeckel 36 abgestützt ist. Zwischen den Zylinderdeckeln 36 und dem äußeren Rohr 10 der Messsonde 2 ist jeweils eine Kolbenstangendichtung 33 und zwischen dem Kolben 40 und dem Zylindermantel 32 ist eine Kolbendichtung 34 angeordnet.

In den Zylinderdeckeln 36 oder in dem Zylindermantel 32 beiderseits des Kolbens 40 sind Anschlüsse 37, 38 für die Zufuhr und Abfuhr von Druckluft vorgesehen. Je nach der Beaufschlagung der Kolbenräume beiderseits des Kolbens 40 mit Druckluft, wird der Kolben 40 und damit die Messsonde 2 innerhalb des Sondenkörpers 1 nach rechts oder nach links bewegt. Hierbei dient das äußere Rohr 10 der Messsonde 2 als Kolbenstange.

Der Vorteil des pneumatischen Antriebes über die beschriebene Kolben-Zylinder-Anordnung liegt darin, dass die Schubkraft mittig angreift. Im Gegensatz zu dem Drehantrieb über den Antriebsmotor 18 und die Zahnstange 17 gemäß Fig. 1 wirkt bei dem pneumatischen Antrieb gemäß Fig. 2 auf die Lagerung der Messsonde nur die Gewichtskraft und nicht zusätzlich noch eine aus dem Drehantrieb resultierende Lagergegenkraft.

Das rückwärtige Ende des Außenmantels 3 ist in einem Lager 20 gelagert. Die äußere Lagerschale des Lagers 20 ist fest in einem Lagerbock 21 auf einem Fahrwerk angeordnet, das in Längsrichtung der Messgassonde verschiebbar ist. Zu diesem Zweck ist auf dem Lagerbock 21 eine Spindelmutter 22 montiert, durch die eine Spindel 23 geführt ist. An die Spindel 23 greift ein nicht gezeigter, herkömmlicher Antrieb an, der die oszillierende Ein- und Ausfahrt der Messgassonde vornimmt.

Auf dem rückwärtigen Ende des Außenmantels 3 ist ein Zahnrad 24 befestigt, in das ein nicht gezeigter, herkömmlicher Drehantrieb eingreift. Der Drehantrieb erzeugt eine oszillierende Drehbewegung der Messgassonde jeweils um 45° nach links und rechts. Diese oszillierende Drehbewegung verhindert ein Ansammeln und Anbacken von Staub auf dem Außenmantel 3 in der Betriebsstellung der Messgassonde.

Durch den Zwischenraum 7 zwischen dem Außenmantel 3 und dem Innenmantel 4 des Sondenkörpers 1 ist eine Spülgasleitung 25 hindurchgeführt. Die Spülgasleitung 25 endet an der Messkammer 11. Das rückwärtige, nach außen geführte Ende der Spülgasleitung 25 ist mit einem Spülgasanschluss 26 versehen. Über diesen Spülgasanschluss 26 wird Spülgas oder Spülluft impulsweise zugeführt, um das Filter 12 periodisch zu reinigen.

In dem Zwischenraum 7 zwischen dem Außenmantel 3 und dem Innenmantel 4 ist zusätzlich ein Gasentnahmerohr 27 unterbracht. Das Gasentnahmerohr 27 steht auf der einen Seite mit einer Entnahmeöffnung 28 in Verbindung, die an der Sondenspitze in dem Außenmantel 3 auf dessen Unterseite angebracht ist. Das aus dem Abschlussdeckel 6 heraustretende Ende des Gasentnahmerohres 27 ist mit einem Anschluss 29 zur Gasentnahme versehen, durch den entnommenes Messgas abgeführt wird.

An dem rückwärtigen, aus dem Sondenkörper 1 herausragenden Ende der Messsonde ist ein Anschuss 30 für die Zufuhr von Druckluft angebracht. Die zugeführte Druckluft wird periodisch oder bei Bedarf in den baulich gegebenen Rinspalt zwischen dem Sondenkörper 1 und der darin eingebauten Messsonde 2 eingeleitet, um den Ringspalt freizublasen.

## Patentansprüche

1. Messgassonde für die In-Situ-Messung von gasförmigen Rauchgasbestandteilen in einer heissen, staubhaltigen Gasatmosphäre bestehend
- aus einem wassergekühlten Sondenkörper (1) mit einem Aussenmantel (3) und einem Innenmantel (4), die an den Enden mit einem Frontdeckel (5) und einem Abschlussdeckel (6) verbunden sind, wobei der Aussenmantel (3) mit Anschlüssen für die Zufuhr und Abfuhr von Kühlwasser versehen ist,
- aus einer innerhalb des Innenmantels (4) des Sondenkörpers (1) angeordneten Messsonde (2), die in dem Innenmantel (4) des Sondenkörpers (1) angeordnet ist und dort axial vor- und rückwärts bewegt wird, und die ein äusseres, vorzugsweise aus Edelstahl bestehendes Rohr (10), und ein innerhalb des äusseren Rohres (10) angeordnetes Innenrohr (14) aufweist, durch das elektrische Leitungen (15) zu einem Anschlussgehäuse (16) geführt sind, das an dem aus dem Sondenkörper (1) herausragenden Ende der Messsonde (2) angebracht ist,
- aus einem mit dem vorderen Ende des äusseren Rohres (10) der Messsonde (2) verbundenen und aus einem Sinterwerkstoff gefertigten, rohrförmigen Filter (12), der eine Messkammer (11) umschliesst, in der Sensoren (13) zur Bestimmung der gasförmigen Rauchgasbestandteile angeordnet sind,
- und aus einem Stössel (31), der vor der Messkammer (11) angeordnet, mit dem Filter (12) verbunden und mit einer Ausstossplatte (19) versehen ist.

2. Messgassonde nach Anspruch 1, **dadurch gekennzeichnet, dass** an das aus dem Sondenkörper (1) herausragende Ende der Messsonde (2) ein Antrieb angreift, der eine oszillierende Bewegung ausführt.

3. Messgassonde nach Anspruch 2, **dadurch gekennzeichnet, dass** der Antrieb aus einer Kolben-Zylinder-Anordnung besteht, deren Kolben (31) auf dem aus dem Sondenkörper (1) herausragenden Teil des äußeren Rohres (10) der Messsonde (2) aufgeklemmt und in einem Zylindermantel (32) axial geführt ist, der beiderseits des Kolbens (31) durch je einen Zylinderdeckel (36) verschlossen und an dem Sondenkörper (1) befestigt ist.

4. Messgassonde nach Anspruch 3, **dadurch gekennzeichnet, dass** beiderseits des Kolbens (31) je eine Dämpfungsfeder (35) vorgesehen ist, die jeweils auf dem Kolben (31) und auf einem der Zylinderdeckel (36) abgestützt ist.

5. Messgassonde nach Anspruch 3 und 4, **dadurch gekennzeichnet, dass** beiderseits des Kolbens (31) in den Zylinderdeckeln (36) oder dem Zylindermantel (32) Anschlüsse (37, 38) für die Zufuhr und Abfuhr von Druckluft vorgesehen sind.

6. Messgassonde nach Anspruch 2, **dadurch gekennzeichnet, dass** der Antrieb aus einer auf dem äußeren Rohr (10) der Messsonde (2) angebrachten Zahnstange (17) besteht, in die ein Zahnrad eines Antriebsmotors (18) eingreift.

7. Messgassonde nach einem der Ansprüche 1 bis 6, **dadurch gekennzeichnet, dass** der Sondenkörper (1) mit einem eine oszillierende Bewegung ausführenden Drehantrieb versehen ist.

8. Messgassonde nach Anspruch 7, **dadurch gekennzeichnet, dass** der Außenmantel (3) des Sondenkörpers (1) in einem Lager (20) auf einem Lagerbock (21) gelagert ist und dass der Außenmantel (3) mit einem Zahnrad (24) versehen ist, an das der Drehantrieb angreift.

9. Messgassonde nach einem der Ansprüche 1 bis 8, **dadurch gekennzeichnet, dass** der Sondenkörper (1) mit einem eine oszillierende Bewegung in axialer Richtung ausführenden Antrieb versehen ist.

10. Messgassonde nach Anspruch 8 und 9, **dadurch gekennzeichnet, dass** auf dem Lagerbock (21) eine Spindelmutter (22) montiert ist, durch die eine Spindel (23) geführt ist, an die der die oszillierende Bewegung ausführende Antrieb angreift.

11. Messgassonde nach einem der Ansprüche 1 bis 10, **dadurch gekennzeichnet, dass** der Kühlwassereinlauf (8) in dem unteren Teil und der Kühlwasserauslauf (9) in dem oberen Teil des Sondenkörpers (1) sich senkrecht gegenüberstehend angeordnet sind.

12. Messgassonde nach einem der Ansprüche 1 bis 11, **dadurch gekennzeichnet, dass** an der Messkammer (11) eine Spülgasleitung (25) endet, die mit einem Anschluss (29) für die impulsweise Zuführung eines Spülgases zur Reinigung des Filters (12) versehen ist.

13. Messgassonde nach einem der Ansprüche 1 bis 12, **dadurch gekennzeichnet, dass** in dem Zwischenraum (7) zwischen Außenmantel (3) und dem Innenmantel (4) des Sondenkörpers (1) ein Gasentnahmerohr (27) angeordnet ist, dessen eines Ende mit einer in dem Außenmantel (3) angebrachten Entnahmeöffnung (28) verbunden ist und dessen anderes Ende aus dem Abschlussdeckel (6) herausragt und mit einem Anschluss (29) versehen ist.

14. Messgassonde nach einem der Ansprüche 1 bis 13, **dadurch gekennzeichnet, dass** an dem rückwärtigen, aus dem Sondenkörper (1) herausragenden Ende der Messsonde (2) ein Anschluss (30) angeordnet ist und dass der Anschluss (30) periodisch oder bedarfsweise mit Druckluft beaufschlagt ist.

## Claims

1. Measuring gas probe for in situ measuring of gaseous flue gas constituents in a hot gas atmosphere containing dust, consisting of
- a water cooled probe body (1) with an outer casing (3) and an inner casing (4), which are connected at the ends by a front cover (5) and a closure cover (6), wherein the outer casing (3) is provided with connections for the feed and discharge of cooling water,
- a measuring probe (2) arranged within the inner casing (4) of the probe body (1), the probe being arranged in the inner casing (4) of the probe body (1) and axially moved forwards and backwards there and comprising an outer tube (10), which preferably consists of stainless steel, and an inner tube (14), which is arranged within the outer tube (10) and through which electrical lines (15) are led to a connecting housing (16), which is mounted at the end of the measuring probe (2) projecting out of the probe body (1),
- a tubular filter (12) which is connected with the front end of the outer tube (10) of the measuring probe (2) and made of a sintered material and which encloses a measuring chamber (11) in which sensors (13) for determining the gaseous flue gas constituents are arranged,
- and a plunger (31) which is arranged in front of the measuring chamber (11) and connected with the filter (12) and which is provided with an ejector plate (19).

2. Measuring gas probe according to claim 1, **characterised in that** a drive which executes an oscillating motion engages at the end of the measuring probe (2) projecting out of the probe body (1).

3. Measuring gas probe according to claim 2, **characterised in that** the drive consists of a piston-cylinder arrangement, the piston (31) of which is clamped on the part of the outer tube (10) of the measuring probe (2) projecting out of the probe body (1) and is axially guided in a cylinder casing (32), which is closed on either side of the piston (31) by a respective cylinder cover (36) and which is fastened to the probe body (1).

4. Measuring gas probe according to claim 3, **characterised in that** a respective damping spring (35) is provided on either side of the piston (31) and is supported on the piston (31) and a respective one of the cylinder covers (36).

5. Measuring gas probe according to claim 3 and 4, **characterised in that** connections (37, 38) for the feed and discharge of compressed air are provided on either side of the piston (31) in the cylinder covers (36) or the cylinder casing (32).

6. Measuring gas probe according to claim 2, **characterised in that** the drive consists of a rack (17) which is mounted on the outer tube (10) of the measuring probe (2) and in which a gearwheel of a drive motor (18) engages.

7. Measuring gas probe according to one of claims 1 to 6, **characterised in that** the probe body (1) is provided with a rotary drive executing an oscillatory motion.

8. Measuring gas probe according to claim 7, **characterised in that** the outer casing (3) of the probe body (1) is mounted in a bearing (20) on a bearing block (21) and that the outer casing (3) is provided with a gearwheel (24) at which the rotary drive engages.

9. Measuring gas probe according to one of claims 1 to 8, **characterised in that** the probe body (1) is provided with a drive executing an oscillatory motion in axial direction.

10. Measuring gas probe according to claim 8 and 9, **characterised in that** mounted on the bearing block (21) is a spindle nut (22) through which a spindle (23) is guided, at which the drive executing the oscillatory motion engages.

11. Measuring gas probe according to one of claims 1 to 10, **characterised in that** the cooling water inlet (8) is arranged in the lower part and the cooling water outlet (9) in the upper part of the probe body (1) to be perpendicularly opposite.

12. Measuring gas probe according to one of claims 1 to 11, **characterised in that** a flushing gas duct (25) ends at the measuring chamber (11) and is provided with a connection (29) for the pulsed feed of a flushing gas for cleaning the filter (12).

13. Measuring gas probe according to one of claims 1 to 12, **characterised in that** a gas removal pipe (27), one end of which is connected with a removal opening (29) formed in the outer casing (3) and the other end of which projects out of the closure cover (6) and is provided with a connection (29), is arranged in the intermediate space (7) between outer casing (3) and the inner casing (4) of the probe body (1).

14. Measuring gas probe according to one of claims 1 to 13, **characterised in that** a connection (30) is arranged at the rearward end of the measuring probe (2) projecting out of the probe body (1) and that the connection (30) is loaded with compressed air periodically or as needed.

## Revendications

1. Sonde de gaz à mesurer pour mesurer in-situ des composants gazeux de gaz de fumées dans une atmosphère gazeuse chaude, chargée en poussières, constituée
- d'un corps de sonde (1) refroidi par eau comportant une enveloppe extérieure (3) et une enveloppe intérieure (4) qui sont reliées aux extrémités à un couvercle frontal (5) et à un couvercle de fermeture (6), l'enveloppe extérieure (3) étant pourvue de raccords pour l'entrée et la sortie d'eau de refroidissement,
- d'une sonde de mesure (2) disposée à l'intérieur de l'enveloppe intérieure (4) du corps de sonde (1), qui est disposée dans l'enveloppe intérieure (4) du corps de sonde (1) et y est déplacée axialement en avant et en arrière, et qui comporte un tube extérieur (10) de préférence constitué d'acier spécial et un tube intérieur (14) disposé à l'intérieur du tube extérieur (10), par lequel des lignes électriques (15) sont amenées à un boîtier de raccordement (16) qui est monté à l'extrémité de la sonde de mesure (2) qui fait saillie hors du corps de sonde (1),
- d'un filtre (12) tubulaire relié à l'extrémité antérieure du tube extérieur (10) de la sonde de mesure (2) et fabriqué en un matériau fritté, qui entoure une chambre de mesure (11) dans laquelle sont disposés des capteurs (13) pour déterminer les composants gazeux de gaz de fumées,
- et d'un piston (31), disposé en amont de la chambre de mesure (11), qui est relié au filtre (12) et pourvu d'une plaque d'éjection (19).

2. Sonde de gaz à mesurer selon la revendication 1, **caractérisée en ce qu'**un entraînement qui exécute un mouvement oscillant, vient en prise à l'extrémité de la sonde de mesure (2) qui fait saillie hors du corps de sonde (1).

3. Sonde de gaz à mesurer selon la revendication 2, **caractérisée en ce que** l'entraînement est constitué d'un dispositif piston/cylindre, dont le piston (31) est emmanché sur la partie du tube extérieur (10) de la sonde de mesure (2) qui fait saillie hors du corps de sonde (1) et est guidé axialement dans une enveloppe de cylindre (32) qui est fermée des deux côtés du piston (31) par chaque fois un couvercle de cylindre (36) et est fixée au corps de sonde (1).

4. Sonde de gaz à mesurer selon la revendication 3, **caractérisée en ce que** des deux côtés du piston (31) est prévu chaque fois un ressort d'amortissement (35) qui est en appui respectivement sur le piston (31) et sur l'un des couvercles de cylindre (36).

5. Sonde de gaz à mesurer selon la revendication 3 et 4, **caractérisée en ce que** des raccords (37, 38) sont prévus des deux côtés du piston (31) dans les couvercles de cylindre (36) ou l'enveloppe de cylindre (32), pour l'entrée et la sortie d'air comprimé.

6. Sonde de gaz à mesurer selon la revendication 2, **caractérisée en ce que** l'entraînement est constitué d'une crémaillère (17) montée sur le tube extérieur (10) de la sonde de mesure (2), sur laquelle vient en prise une roue dentée d'un moteur d'entraînement (18).

7. Sonde de gaz à mesurer selon l'une des revendications 1 à 6, **caractérisée en ce que** le corps de sonde (1) est pourvu d'un entraînement rotatif exécutant un mouvement oscillant.

8. Sonde de gaz à mesurer selon la revendication 7, **caractérisée en ce que** l'enveloppe extérieure (3) du corps de sonde (1) est supportée dans un palier (20) sur un support de palier (21) et que l'enveloppe extérieure (3) est pourvue d'une roue dentée (24) sur laquelle vient en prise l'entraînement rotatif.

9. Sonde de gaz à mesurer selon l'une des revendications 1 à 8, **caractérisée en ce que** le corps de sonde (1) est pourvu d'un entraînement exécutant un mouvement oscillant dans la direction axiale.

10. Sonde de gaz à mesurer selon la revendication 8 et 9, **caractérisée en ce que** sur le support de palier (21) est monté un écrou de broche (22) à travers lequel passe une broche (23) sur laquelle vient en prise l'entraînement exécutant le mouvement oscillant.

11. Sonde de gaz à mesurer selon l'une des revendications 1 à 10, **caractérisée en ce que** l'entrée d'eau de refroidissement (8) dans la partie inférieure et la sortie d'eau de refroidissement (9) dans la partie supérieure du corps de sonde (1) sont disposées verticalement en se faisant mutuellement face.

12. Sonde de gaz à mesurer selon l'une des revendications 1 à 11, **caractérisée en ce qu'**à la chambre de mesure (11) se termine une conduite de gaz de rinçage (25) qui est pourvue d'un raccord (29) pour l'alimentation par impulsion d'un gaz de rinçage destiné au nettoyage du filtre (12).

13. Sonde de gaz à mesurer selon l'une des revendications 1 à 12, **caractérisée en ce que** dans l'espace intermédiaire (7) entre l'enveloppe extérieure (3) et l'enveloppe intérieure (4) du corps de sonde (1) est disposé un tube de prélèvement de gaz (27) dont l'une des extrémités est reliée à une ouverture de prélèvement (28) ménagée dans l'enveloppe extérieure (3) et dont l'autre extrémité fait saillie hors du couvercle de fermeture (6) et est pourvue d'un raccord (29).

14. Sonde de gaz à mesurer selon l'une des revendications 1 à 13, **caractérisée en ce qu'**un raccord (30) est disposé à l'extrémité arrière de la sonde de mesure (2) qui fait saillie hors du corps de sonde (1) et que le raccord (30) reçoit de l'air comprimé périodiquement ou selon les besoins.
